(19) Europäisches Patentamt
European Patent Office
Office européen des brevets

(11) **EP 3 264 070 A1**

(12) **EUROPEAN PATENT APPLICATION**

(43) Date of publication:
03.01.2018 Bulletin 2018/01

(51) Int Cl.:
*G01N 21/77* (2006.01)     *G01N 21/35* (2014.01)
*G01N 21/552* (2014.01)     *G01N 21/65* (2006.01)

(21) Application number: 17178959.7

(22) Date of filing: 30.06.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 30.06.2016 US 201662356697 P

(71) Applicant: Sightline Innovation Inc.
Toronto, Ontario M6H3A7 (CA)

(72) Inventors:
• TRENHOLM, Wallace
Toronto, Ontario M5C1S3 (CA)
• MAVINKURVE, Maithili
Toronto, Ontario L3P7R9 (CA)
• CASSIDY, Jason
Winnipeg, Manitoba R3C1J8 (CA)

(74) Representative: TBK
Bavariaring 4-6
80336 München (DE)

(54) **WAVEGUIDE-BASED SYSTEM AND METHOD FOR BIOMARKER DETECTION**

(57) Systems, methods, and modules for detecting a biomarker in a sample are described. A system for detecting presence or absence of a biomarker in a sample includes: a light source for producing electromagnetic radiation for interrogating the sample; a biosensor module including: a waveguide for guiding the electromagnetic radiation, the waveguide exposed to the sample; and a recognition element affixed to the waveguide and configured to bind to the biomarker; a detector for receiving the electromagnetic radiation from the waveguide and detecting a signal corresponding to an interaction of the electromagnetic radiation with the biomarker bound to the recognition element, in accordance with at least one detection modality; and a computing device for analyzing data related to the signal in order to detect presence or absence of the biomarker in the sample.

FIG. 1

**Description**

FIELD

The present disclosure relates to biomarker detection. More particularly, the present disclosure relates to a system, method, and module for detecting presence or absence of a biomarker in a sample.

BACKGROUND

Systems capable of detecting the presence or level of a biomarker in a sample are desirable when trying to diagnose or identify a condition of interest in a subject. This is because in many instances biomarkers are known to indicate the presence or absence of a particular disease, pathogen or physiological state. Systems and processes relying on biomarker detection are becoming increasingly important in promoting the health of individuals and populations in today's modern world due to such factors as improved and increasingly accessible long-distance travel methods. This makes the need for quick, accurate, and non-invasive detection of biomarkers to prevent the spread of disease critical.

Traditional methods for detecting biomarkers have significant limitations. Processes such as ELISA, PCR, and culturing, can be time-consuming and, in some circumstances, require additional processing and labelling steps that further slow or complicate the process. Many of these traditional techniques also require lab personnel or other human intervention throughout or during certain aspects of the process. Equipment is typically not easily portable and lab resources may be required. Such limitations can make the scaling of current techniques for rapid detection impractical, and can prevent their efficient implementation in isolated areas and locations with reduced infrastructure.

While many current techniques are valued for their sensitivity and specificity, the requirement for relatively large sample sizes from the subject can prove inconvenient, invasive, slow, etc. Further, many existing methods cannot be effectively multiplexed and may require a number of independent tests to determine the presence of different biomarkers, making them ineffective candidates for rapid, point-of-care detection of multiple biomarkers.

Accordingly, it is desired to have an improved system and method for detecting presence or absence of a biomarker in a substance that overcomes at least some of the problems of conventional biomarker detection techniques.

SUMMARY

These and other aspects are contemplated and described herein. It will be appreciated that the foregoing summary sets out representative aspects of systems, methods, modules for biomarker detection to assist skilled readers in understanding the following detailed description.

In an aspect, there is provided a system for detecting presence or absence of a biomarker in a sample comprising: a light source for producing electromagnetic radiation for interrogating the sample; a biosensor module comprising: a waveguide for guiding the electromagnetic radiation, the waveguide exposed to the sample; and a recognition element affixed to the waveguide and configured to bind to the biomarker; a detector for receiving the electromagnetic radiation from the waveguide and detecting a signal corresponding to an interaction of the electromagnetic radiation with the biomarker bound to the recognition element, in accordance with at least one detection modality; and a computing device, having one or more processors, for analyzing data related to the signal in order to detect presence or absence of the biomarker in the sample.

In a particular case, the system further comprises a microfluidic device for effecting movement of the sample through the biosensor module.

In another case, the system further comprises a filtration system for filtering the sample according to at least one physical characteristic.

In yet another case, at least one of the physical characteristics is sizes of molecules in the sample.

In yet another case, at least one of the detection modalities is an interferometry modality for generating an interference pattern as the signal.

In yet another case, at least one of the detection modalities is selected from the group consisting of Surface-Enhanced Raman Spectroscopy (SERS), Surface Plasmon Resonance (SPR), Surface Plasmon Resonance Imaging (SPRi), Localised Surface Plasmon Resonance (LSPR), Optofluidic Nanoplasmonic, Optical waveguide-based sensing, Optical ring resonator-based sensing, Photonic crystal-based sensing, Nanosensitive Optical Coherence Tomography (OCT) sensing, Lensless digital holographic imaging, Superresolution microscopy techniques, piezoelectric sensing, nano-cantilever sensing, Raman spectroscopy (RS), Resonance Raman spectroscopy (RRS), and infrared spectroscopy (IRS).

In yet another case, the computing device comprises a neural network for receiving the data related to the signal at an input layer and generating the determination of the presence or absence of the biomarker in the sample at an output layer.

In yet another case, data received at the input layer further comprises at least one of protein interaction data, nucleic acid data, biomarker identification data, genomic sequencing data, mass spectrometry data, time series genomic data, and medical history data.

In yet another case, the biosensor module comprises a plurality of cells, each cell comprising a separate waveguide and recognition element.

In yet another case, the waveguide couples the electromagnetic radiation using a coupling approach selected from the group consisting of front-face coupling, prism coupling, and grating coupling.

In another aspect, there is provided, a method of detecting presence or absence of a biomarker in a sample, comprising: exposing a waveguide to the sample and binding the biomarker via a recognition element affixed to the waveguide; producing electromagnetic radiation directed at the waveguide, the waveguide guiding the electromagnetic radiation towards a detector; receiving the electromagnetic radiation at the detector; detecting a signal at the detector corresponding to the interaction of the electromagnetic radiation with the biomarker bound to the recognition element, in accordance with at least one detection modality; and determining presence or absence of the biomarker, by a computing device having one or more processors, by analyzing data related to the signal.

In a particular case, the method further comprises filtering the sample according to at least one physical characteristic.

In another case, at least one of the physical characteristics is sizes of molecules in the sample.

In yet another case, at least one of the detection modalities is an interferometry modality for generating an interference pattern as the signal.

In yet another case, at least one of the detection modalities is selected from the group consisting of Surface-Enhanced Raman Spectroscopy (SERS), Surface Plasmon Resonance (SPR), Surface Plasmon Resonance Imaging (SPRi), Localised Surface Plasmon Resonance (LSPR), Optofluidic Nanoplasmonic, Optical waveguide-based sensing, Optical ring resonator-based sensing, Photonic crystal-based sensing, Nanosensitive Optical Coherence Tomography (OCT) sensing, Lensless digital holographic imaging, Superresolution microscopy techniques, piezoelectric sensing, nano-cantilever sensing, Raman spectroscopy (RS), Resonance Raman spectroscopy (RRS), and infrared spectroscopy (IRS).

In yet another case, determining presence or absence of the biomarker comprises using a neural network for receiving the data related to the signal at an input layer and generating the determination of the presence or absence of the biomarker in the sample at an output layer.

In yet another case, the neural network is a convolutional feed-forward neural network.

In yet another case, data received at the input layer further comprises at least one of protein interaction data, nucleic acid data, biomarker identification data, genomic sequencing data, mass spectrometry data, time series genomic data, and medical history data.

In yet another case, the waveguide couples the electromagnetic radiation using a coupling approach selected from the group consisting of front-face coupling, prism coupling, and grating coupling.

In another aspect, there is provided a biosensor module for use in a system for detecting a biomarker in a sample, the system comprising a light source for producing electromagnetic radiation and a detector for receiving the electromagnetic radiation and detecting a signal corresponding to an interaction of the electromagnetic radiation with the biomarker, the biosensor module comprising: a first plate; a second plate; a waveguide for guiding the electromagnetic radiation and positioned between the first and second plates, the waveguide having a first portion integral with the first plate and a second portion integral with the second plate; and a recognition element affixed to a portion of the waveguide, the recognition element configured to bind to the biomarker.

Other features and advantages of the present invention are described more fully below.

BRIEF DESCRIPTION OF THE DRAWINGS

The features of the invention will become more apparent in the following detailed description in which reference is made to the appended drawings wherein:

FIG. 1 is a block diagram of a system for biomarker detection in accordance with an embodiment;

FIG. 2 shows a collection method of whole saliva;

FIG. 3 shows a microfluidic device comprising a micro-pump infusion system for use with a biomarker detection system, in accordance with an embodiment;

FIG. 4 shows a self-powered microfluidic device via degassing driven flow for use with a biomarker detection system, in accordance with an embodiment;

FIG. 5 shows a movable 3D printed microfluidic device for use with a biomarker detection system, in accordance with an embodiment;

FIG. 6 shows a cascading filtration system for use with the biomarker detection system of FIG. 1, in accordance with an embodiment;

FIG. 7 shows an individual cell of a multiplexed biosensor module, in accordance with an embodiment;

FIG. 8 shows a silicon nanowire surface modification chemistry and bioreceptor functionalization, in accordance with an embodiment;

FIG. 9 shows an exploded view of a biosensing element chip, in accordance with an embodiment;

FIG. 10 is a schematic diagram of a biosensor module having a plurality of biosensing elements, in accordance with an embodiment;

FIG. 11 shows a method of fabricating a nanowire waveguide with a grating coupler, in accordance with an embodiment; and

FIG. 12 is a schematic diagram of a computing module for use in a biomarker detection system, in accordance with an embodiment.

## DETAILED DESCRIPTION

Before the subject matter of the present disclosure is described in further detail, it is to be understood that the invention is not limited to the particular embodiments described, as such may, of course, vary. It is also to be understood that the terminology used herein is for the purpose of describing particular embodiments only, and is not intended to be limiting, since the scope of the present invention will be limited only by the appended claims.

For simplicity and clarity of illustration, where considered appropriate, reference numerals may be repeated among the Figures to indicate corresponding or analogous elements. In addition, numerous specific details are set forth in order to provide a thorough understanding of the embodiments described herein. However, it will be understood by those of ordinary skill in the art that the embodiments described herein may be practiced without these specific details. In other instances, well-known methods, procedures and components have not been described in detail so as not to obscure the embodiments herein. Also, the description is not to be considered as limiting the scope of the embodiments described herein.

Various terms used throughout the present disclosure may be read and understood as follows, unless the context indicates otherwise: "or" as used throughout is inclusive, as though written and/or; singular articles and pronouns as used throughout include their plural forms, and vice versa; similarly, gendered pronouns include their counterpart pronouns so that pronouns should not be understood as limiting anything described herein to use, implementation, performance, etc. by a single gender; "exemplary" should be understood as "illustrative" and "exemplifying" and not necessarily as "preferred" over other embodiments. Further definitions for terms may be set out herein; these may apply to prior and subsequent instances of those terms, as will be understood from a reading of the present disclosure/description.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, a limited number of the exemplary methods and materials are described herein.

It must be noted that as used herein and in the appended claims, the singular forms "a", "an", and "the" include plural referents unless the context clearly dictates otherwise.

Any module, unit, component, server, computer, terminal, engine, or device exemplified herein that executes instructions may include or otherwise have access to computer readable media such as storage media, computer storage media, or data storage devices (removable and non-removable) such as, for example, magnetic discs, optical disks, or tape. Computer storage media may include volatile and non-volatile, removable and non-removable media implemented in any method or technology for storage of information, such as computer readable instructions, data structures, program modules, or other data. Examples of computer storage media include RAM, ROM, EEPROM, flash memory or other memory technology, CD-ROM, digital versatile discs (DVD) or other optical storage, magnetic cassettes, magnetic tape, magnetic disk storage or other magnetic storage devices, or any other medium which can be used to store the information and which can be accessed by an application, module, or both. Any such computer storage media may be part of the device or accessible or connectable thereto. Further, unless the context clearly indicates otherwise, any processor or controller set out herein may be implemented as a singular processor or as a plurality of processors. The plurality of processors may be arrayed or distributed, and any processing function referred to herein may be carried out by one or by a plurality of processors, even though a single processor may be exemplified. Any method, application or module herein described may be implemented using computer readable/executable instructions that may be stored or otherwise held by such computer readable media and executed by the one or more processors.

The following relates generally to biomarker detection and more particularly a system, method, and module for detecting presence or absence of a biomarker in a sample.

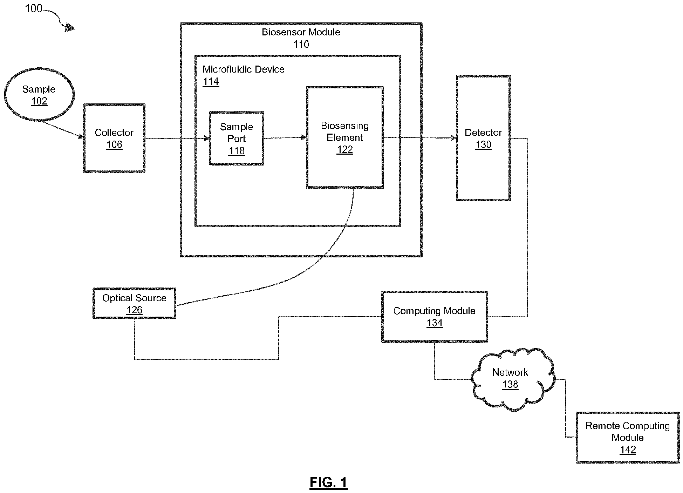

Referring now to FIG. 1, shown therein is a system 100 for detecting a biomarker in a sample, in accordance with an embodiment. The detection of the presence or absence of a biomarker by the system 100 can be used to indicate the presence or absence of a condition (e.g. disease) in a subject providing the sample. Generally, the system 100 includes a biosensor module 110, an optical source 126, a detector 130, and a computing module 134. In some embodiments, components of the system 100, such as biosensor module 110, may take the form of a biosensor chip. A sample 102 is obtained from a subject and transferred to a collector 106. The collector 106 can be any collector suitable for receiving and potentially storing the sample 102. In some cases, the collector 106 can be connected to, or form part of, the biosensor module 110. The sample 102 is transferred from the collector 106 to a sample port 118 on the biosensor module 110. Once received by the sample port 118, movement of the sample 102 through the biosensor module 110 can be effected by a microfluidic device 114, to allow necessary interactions. The biosensor module 110 further comprises a biosensing element 122 which, when exposed to sample 102, causes an interaction with target biomarker present in the sample 102 such that the biomarker binds to a portion of the bionsensing element 122. In some cases, the biosensor module 110 may include a filtration element for filtering the sample 102 prior to exposure to the biosensing element 122. In an embodiment, the bionsensing element 122 comprises a waveguide having a recognition element affixed to the waveguide, the recognition element configured to bind the target biomarker. After exposure to the sample 102, electromagnetic radiation is output via optical source 126 to biosensing element 122 and propagated along a length of biosensing element 122 to detector 130. Biosensor module 110 may include input and output coupling for transmitting the electromagnetic radiation to and from the biosensing element 122 and other components of system 100. The electromagnetic radiation received by the detector 130 comprises an optical signal, as the presence of biomarker bound to the biosensing element 122 can influence the behavior of the electromagnetic radiation. Accordingly, the optical signal may contain information about the presence of the biomarker in the sample 102, and thus the condition (e.g. disease state) of the subject from which the sample was taken. In some cases, the optical signal may comprise an interference pattern, such as in embodiments where system 100 implements interferometry. In such an embodiment, optical source 126 may be any optical source suitable for use with an interferometric type modality, such as a laser or light emitting diode (LED), and biosensing element 122 may comprise an interferometer-type configuration for generating an interference pattern. The detector 130 generates a detection signal from the received electromagnetic radiation and transmits the detection signal to computing module 134 for processing and analysis. Analysis may include comparing the detection signal (or data derived therefrom) with a reference signal or application of machine learning techniques to data related to the detection signal.

The computing module 134 may be operable to connect to one or more remote computing modules 142 through a network 138, which can be a personal, local or wide area network, such as the Internet. In variations, the system 100 may include only a remote computing module 142.

The system 100 can be portable or fixed; for example, in some implementations, the system 100 may be transportable to different locations, such as for deployment in field operations, or may be a fixed system, such as for use in hospitals, universities, research facilities, doctors' offices, labs, and the like.

Various detection modalities are contemplated for use with system 100. For example, the system 100 may comprise a molecular sensing array designed to be sensitive to one or more target biomarkers. Further, components of system 100 may be configured to perform Surface-Enhanced Raman Spectroscopy (SERS), Surface Plasmon Resonance (SPR), Surface Plasmon Resonance Imaging (SPRi), Localised Surface Plasmon Resonance (LSPR), Optofluidic Nanoplasmonic, Optical waveguide-based sensing, Optical ring resonator-based sensing, Photonic crystal-based sensing, Nanosensitive Optical Coherence Tomography (OCT) sensing, Lensless digital holographic imaging, Superresolution microscopy techniques, piezoelectric sensing, nano-cantilever sensing, Raman spectroscopy (RS), Resonance Raman spectroscopy (RRS), and infrared spectroscopy (IRS). In variations, the system 100 can be configured to perform interferometry-based detection, such as by using a Mach-Zehnder Interferometer, Michelson-type interferometer, Fabry-Perot interferometer, Young's interferometer, Hartman interferometer, Interferometric scattering microscopy (iSCAT), Single Particle Interferometric Reflectance Imaging (SPIRIS) and backscattering interferometry. Other detection techniques will now occur to a person of skill and are contemplated.

In some variations, the system 100 may use multiple different detection techniques and/or variations in a particular detection technique in combination, thus allowing system 100 to take advantage of the fact that different detection techniques and/or variations in the same technique can yield similar but complementary information, potentially increasing the detectability of the biomarker in the sample 102. The different techniques and/or variations in the detection techniques can be performed simultaneously, sequentially, or a combination of both. For example, in some embodiments, system 100 can include appropriate components to perform variations of the same detection technique. For example, system 100 can include multiple optical sources 126, such as multiple fixed wavelength lasers capable of generating source radiation at different wavelengths. Alternatively, optical source 126 may comprise a single tunable radiation source such as a tunable laser that can generate source radiation at different wavelengths. In such cases, the same spectroscopy technique can be performed at various wavelengths on sample 102. As a further example of varying a detection technique, the same detection technique can be performed at different temperatures. For example, the system 100 can include a heating element such as an infrared laser operable to change the temperature of the sample 102.

In some variations, the different detection techniques and/or variations of the same detection technique can be applied to processed and/or unprocessed samples. For example, a plurality of detection techniques or variations of the same detection technique can be utilized by system 100 on sample 102. In some cases, to perform multiple detection techniques or multiple variations of a detection technique, components of system 100 may be multiplicated as needed (e.g. multiple detectors 130, multiple biosensor modules 110, etc.).

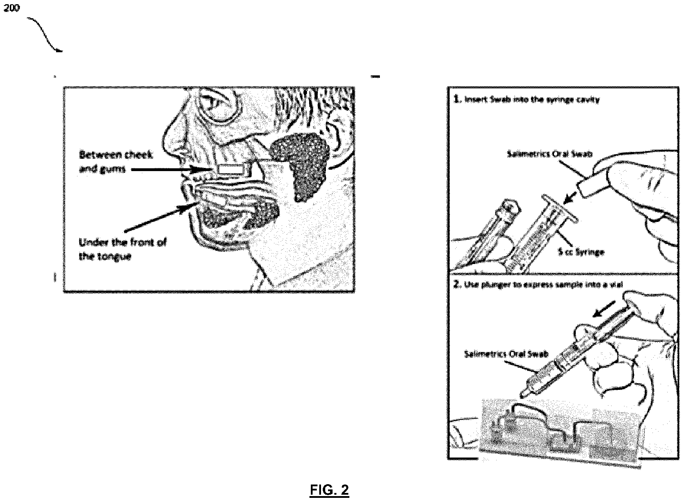

Sample 102 may comprise any biological sample which may contain the target biomarker. Sample 102 may be a fluid sample such as saliva, urine, whole blood, plasma, semen, vaginal, peritoneal, cerebrospinal, stool, etc., taken from a subject. The biomarker may comprise any molecule that is capable of indicating the presence of a particular condition in the subject; for example, the biomarker may be a protein antigen (e.g. pathogen-related), a nucleic acid (e.g. DNA, RNA, miRNA), an antibody (such as one detectable in human or animal host bodily fluids), a heavy metal, a POP, an EDC, a toxin, a virus, or other human and/or animal disease-related biomarker. The sample 102 may be provided by a subject who is to be tested for a specific biomarker which may, in some cases, be found in saliva or other various bodily fluids, such as a subject's blood, semen, urine, stool, cerebrospinal fluid, breast milk, vaginal, peritoneal, and other mucosal secretions. In an illustrative example, the sample 102 comprises saliva and can be collected using an oral swab technique as shown in FIG. 2. The collected whole saliva is transferred into the sample port 118 which contains a pre-loaded phosphate buffered saline (PBS) in a 1:4-1:100 dilution. This collection procedure can be repeated if additional volume of the sample 102 is required. In alternative embodiments, standard collection methods for bodily fluid samples (as mentioned above) as known in the art can be employed.

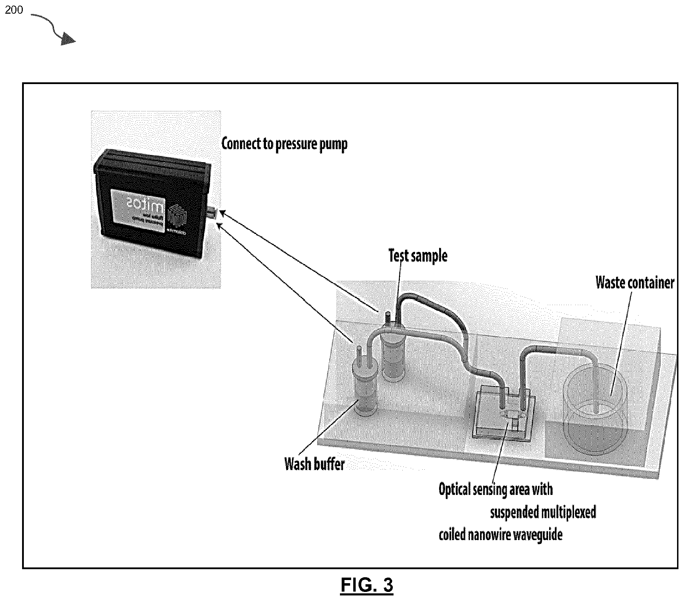

In some cases, the microfluidic device 114 comprises a microfluidic pump for propelling the sample 102 through the biosensor module 110. Referring now to FIG. 3, a lab-on-chip microfluidic system 300 is shown, in accordance with an embodiment. The system 300 comprises a microfluidic device. In an embodiment, movement of sample 102 is effected by an off-chip micropump system connected to on-chip tubing.

The microfluidic device can be fabricated by directly printing out the microfluidic device using a 3D printer or similar technology. In an embodiment, the material of the microfluidic device comprises clear 3D printing resin (e.g. Milcraft). The printed microfluidic device may then be bonded to a substrate of the biosensor module 110 using an appropriate adhesive, such as a UV sensitive adhesive glue.

In an alternate embodiment, the microfluidic device 114 can be fabricated by first printing out a master using a 3D printer. The master may then be used to mold the microfluidic device 114, which may, in some implementations, comprise polydimethylsiloxane (PDMS). After curing, the microfluidic device 114 is peeled off from the master. Holes may be punched in the microfluidic device 114 for use as an inlet and outlet. The inlet of the microfluidic device may be connected to one or more containers (e.g. glass vial bottles) holding a test sample or a wash buffer. In variations, this connection may be facilitated by polyethylene or similarly appropriate tubing. Tubing may also be used to connect the containers to a low-pressure pump, which can generate desirable pressure to drive the sample 102 into the microfluidic device 114. The outlet of the microfluidic device 114 may be connected to a disposal container.

In a particular embodiment, the microfluidic device 114 can be operated by: infusing the washer buffer; opening the seal cap and expelling the 1-2mL of whole saliva into the sample port with the dilution buffer; closing the seal cap and connecting the tubing to the micro air compressor; turning on a micro air compressor or pump and infusing the saliva sample into the biosensor module 110; turning off the micro air compressor and allowing a reaction to take place; turning on the micro air compressor to infuse the washing buffer from the buffer port; and turning off the micro air compressor and detecting a signal.

In alternative embodiments, the microfluidics device 114 can be self-powered (e.g. degassing driven flow) or a movable 3D printed system, or a combination of two or more of a micro-pump infusion system, self-powered system, and a movable 3D printed system.

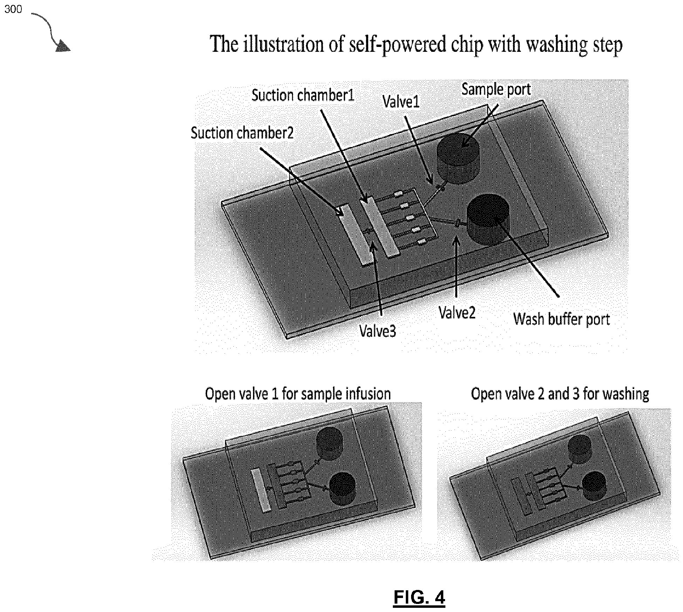

Referring now to FIG. 4, an alternative embodiment having a self-powered microfluidic system 400 using degassing driven flow is shown. Sample flow is propelled with a self-priming degassing-driven flow technique by degassing the PDMS channels in a standard vacuum desiccator, vacuum sealing the microfluidic device.

In an embodiment, the system 400 can be operated by: mixing saliva or other sample with buffer in the sample port; opening a first valve, allowing a first suction chamber to perform sample infusion; once the first suction chamber is filled and a reaction occurs, closing the first valve, and opening a second and third valve, allowing a second suction chamber to start performing a washing step. Once the second chamber is filled, a detection operation can be performed on the sample.

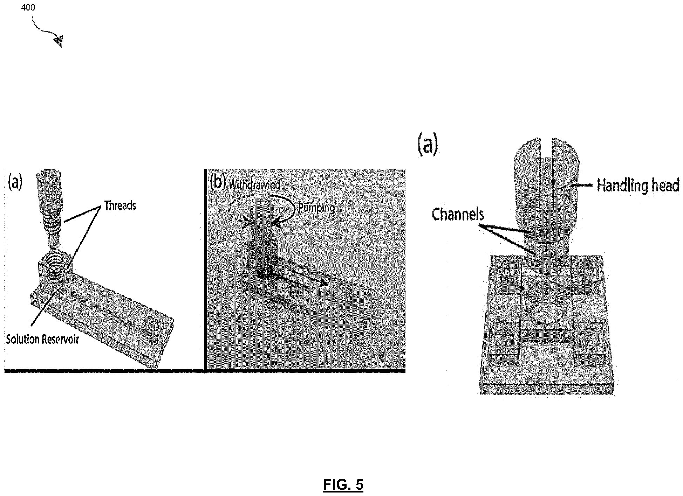

Referring now to FIG. 5, an alternative embodiment of microfluidic device 114 comprising a movable 3D printed microfluidic system is shown. Sample flow may be propelled via a torque-actuated pump, which may further comprise push valves. The flow of sample 102 and washer buffer may be controlled via a rotary valve.

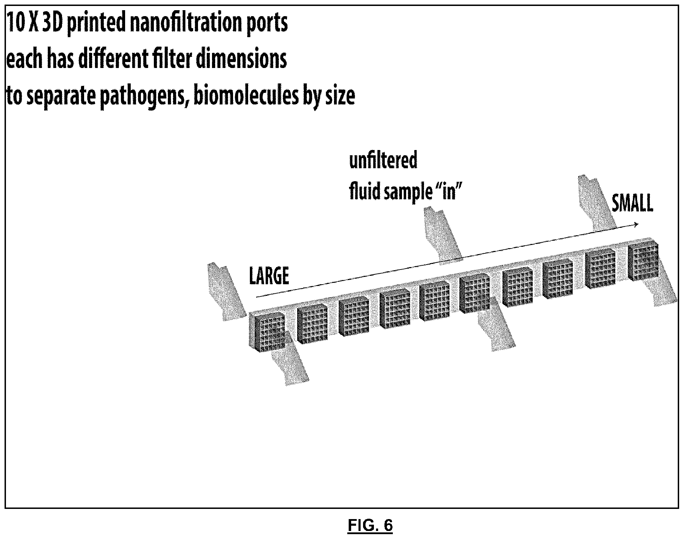

The biosensor module 110 may include a filtration device for selectively filtering the sample 102 according to at least one physical characteristic, such as size. As an example, FIG. 6 shows a filtration device comprising a nanofilter for separating components in the sample 102 (e.g. pathogens, biomolecules, biomarkers, other molecules etc.) on the basis of size, in accordance with an embodiment. The nanofilter may be incorporated as part of the microfluidic device 114. The nanofilter may comprise a plurality of cascading nanofilters having various dimensions suitable for separating molecules of various sizes in the sample 102. In an embodiment, the microfluidic device 114 effects movement of the sample 102 through the nanofilter prior to exposing the sample 102 to the biosensing element 122. The nanofilter may limit non-specific binding to the recognition element by filtering sample components based on size to specific channels.

In an alternative embodiment, the sample 102 moves downstream through different sized nanofilters (e.g. large to small). The filtered sample is then delivered laterally to specific channels to the biosensing element for interaction.

In variations, the filtration device can be fabricated by 3D printing via Direct Laser Writing (DLW) (e.g. two-photon polymerization (2PP)), nanoimprinting lithography, or other similar methods to directly print with photoresistant polymers the final shape parts onto pre-structured substrates. This can facilitate the fabrication of numerous microfluidic filters on a microfluidic chip.

In an embodiment, optical source 126, which can be a frequency swept laser beam (e.g. at -1550nm), outputs electromagnetic radiation to the biosensor module 110 as a free space p-/s-polarized beam at a calculated incident angle (dependent on grating parameters and the RI of grating material) onto an input grating coupler. The electromagnetic radiation emitted by the optical source 126 output fiber may be first collimated and then focused onto the input grating coupler on the first plate of the biosensor module 110, such as by using a pair of achromatic lenses with appropriate focal lengths. Another pair of achromatic lenses may be used to capture the array of beams emitted from the output coupler array of the biosensor module 110 from one side of a second plate, and project the array of beams onto the detector 130 to form an image of the output coupler array.

In some cases, the optical source 126 can include a collimator for narrowing the output beam. In further cases, further optics may be included in various stages of the system 100 to control or change the optical beams. Optics may include lenses or other optical devices suitable to control, guide, navigate, position, or the like, the light beam in a desired manner.

In further cases, software techniques may be employed for correcting or affecting optical errors or signals.

In an embodiment, freely-propagating light from optical source 126 is coupled into a waveguide of biosensing element 122, and the direction of incoupling is controlled. There are various methods of coupling light from optical source 126 into the waveguide that may be utilized in doing this, such as front-face coupling, prism coupling, and grating coupling, among others. Front-face coupling may suffer from low robustness against vibrations in the biosensor module 110, and can require extensive alignment procedures to minimize variations in the coupling efficiency. Embodiments comprising a silicon nanophotonic waveguide may be constrained by the large inherent size difference between comparatively large optical fibers and much smaller photonic waveguides, which may cause an unacceptable amount of loss without a mode size conversion solution. For example, the large inherent size mismatch between the 10.4um typical mode diameter of the light mode in fiber and a 550nm by 225nm rectangular silicon nanowire waveguide means that an enormous amount of power may be lost when trying to move the signal directly from fiber to the waveguide end. Prism coupling to the waveguide may suffer from not being suitable for use in system 100 outside the laboratory, as the pressure of the prism on the waveguide may need to be reproduced with high precision.

In embodiments using grating coupling, the fabrication of a defined grating structure in the waveguide may be necessary, which can be technically more elaborate than embodiments employing prism or front-face coupling. The grating structure allows only a very narrow range of coupling angles, depending on the spectral bandwidth of the optical source 126. The position of the light beam on the grating may influence the coupling efficiency to a large extent, along with grating parameters, such as structure depth. As with prism coupling, the coupling angle may be determined by the effective refractive index of the waveguide (sensing principle of the grating coupler sensor). This technique may have various advantages over methods, such: only the coupling angle on the incident beam needs to be adjusted in order to achieve the phase matching condition, making this coupling technique simpler to implement; contrary to the prism coupler light can be coupled via the substrate, besides via the cover. This may eliminate the problem that exists in prism coupling configuration, where light is obstructed from fluidic chamber placed on the cover of the waveguide. Additionally, no immersion oil is needed in this configuration and there is good reproducibility of coupling conditions as no further optical elements are directly involved. Some drawbacks include that the production of the waveguide gratings is technologically intensive, and the waveguide gratings can be very sensitive to mechanical vibrations, since the coupling efficiency is very sensitive to the angle of incidence. A grating coupler can be compared to a Bragg grating optimized to diffract light from a free space optical source 126 into a dielectric waveguide. Similarly, an output coupler can also be used to diffract light from a waveguide into a free space detector.

In an embodiment, the biosensing element 122 comprises a transducer, such as a waveguide, that relates the interaction of the recognition element and the target biomarker to a readable optical signal. The term "waveguide", as used herein, may refer to but is not limited to a structure, such as a dielectric insulating medium-to-high refractive index materials (e.g. Si, TiO2), that is able to confine and guide electromagnetic waves. The waveguide may be a material having a refractive index sufficiently high compared to the ambient medium, and appropriate dimensions to guide the light source at distinctive wavelengths by total internal reflection. Total internal reflection can be understood as a phenomenon which occurs when a propagating wave strikes a medium boundary at an angle larger than a particular critical angle ($\theta$c) with respect to the normal to the surface ($\theta > \theta$c) and the entire ray reflects from the boundary with none of the light passing through. Total internal reflection may result in the presence of an evanescent wave beyond the boundary surface. In such an instance, even though the entire incident wave is reflected back into the originating medium, there is some penetration into the second medium at the boundary. The evanescent wave appears to travel along the boundary between the two materials.

The waveguide sensor may rely on the perturbation of the evanescent field of a guided mode caused by optical absorptions, fluorescence or refractive index changes of the measured sample. In an embodiment, refractive index waveguide sensors are utilized because of their easy realization, and the potential for real-time monitoring with a minimal sample volume. The binding of molecules to the waveguide surface produces a change in the effective index Neff of the guided optical mode propagating through the waveguide. This effective index change $\delta$Neff is a result of the coupling of the mode evanescent field with the molecules near the waveguide surface, which causes Neff to vary linearly with the density of molecules at the surface. For a sensor waveguide of length L, the total induced phase shift in response to the effective index perturbation $\delta$Neff is: $\delta\varphi = \delta$Neff L.

A change of the refractive index in the sensing area of the waveguide, generated by biochemical interactions between the target biomarker and the recognition element affixed to the waveguide, can change the effective refractive index of the guided mode, which may include a phase difference between the light beams travelling in both sample and reference arms of the waveguide. Such phase difference can result in an intensity variation at the output of the device that can be expressed as (Eq. (1)):

$$I_T = I_S + I_R + 2\sqrt{I_S I_R} \cos\left[\Delta\varphi_S\left(t\right)\right]$$

where $I_S$ and $I_R$ are the intensities of the light in the sensor and reference arms, respectively. The term $\varphi_S$ is the phase difference between the light beams travelling in sensor and reference arms, and is given by:

$$\Delta\varphi_S\left(t\right) = 2\pi\frac{L}{\lambda}\left(N_S\left(t\right) - N_R\right)$$

where $N_S$ and $N_R$ are the effective refractive indexes of the guided modes in the sensor and reference arms, respectively, $\lambda$ is the wavelength of light and L is the length of the sensing area. As it can be deduced from Eq. (1), the output intensity of the biosensing element is periodic with respect to the phase changes induced in the sensing area. Because the effective refractive index depends on the propagating light wavelength, a phase change between the arms of the biosensing element can be induced by a change in the wavelength of the optical source 126. The introduction of a small change (few nm) in the wavelength of the guided light can produce a variation of the phase difference given by:

$$\delta\left(\Delta\varphi_S\right) = \frac{2\pi}{\lambda}\left[-\frac{1}{\lambda}\left(N_S - N_R\right) + \frac{\partial\left(N_S - N_R\right)}{\partial\lambda}\right]L\delta\lambda$$

When the concentration of the target biomarker on the waveguide surface changes (i.e. via binding to the recognition element), the effective refractive index of the optical waveguide changes and consequently a phase shift will be introduced. This phase shift could be converted into an intensity change or a frequency shift using interferometers or resonant structures. This may include various integrated optical sensors based on different structures and mechanisms, such as Mach-Zehnder interferometers (MZI), and high-Q optical microcavities (including microri ngs/m icrodisks).

Nanophotonic devices for biosensing such as those described herein mainly exploit two different working principles to obtain a transduction signal: change in the refractive index ($\Delta$n), and confinement and enhancement of the electromagnetic field below the diffraction limit. In a particular embodiment, based on the former principle, interferometric and resonant cavity based devices are coated with a recognition element such as an antibody and a binding event between the target biomarker and the recognition element causes a local change in the refractive index. As an example, Young and Mach-Zehnder interferometers can yield limits of detection (LODs) down to $10^{-7}$, but may require a long interaction length in order to aggregate a detectable $\Delta n$. Interferometry techniques incorporate a principle of detection whereby a guided wave undergoes a phase change as its evanescent field interacts with a sample. The most notable design constraint of interferometric devices is that in order to produce a sensitive device, a long interaction length between guided wave and the sample 102 may be necessary. Mach-Zehnder interferometers traditionally incorporate single frequency, single polarization light from optical source 126 that enters a single-mode input waveguide that is split equally at a Y-junction. One branch has a window over the top of it allowing the evanescent field of that branch to interact with the sample 126 while the reference arm is protected from the sample with a cladding layer of an appropriate thickness. The two arms recombine at the output arm, resulting in interference the intensity of which can be measured by detector 130. Generally, the waveguide structure may be single polarization and single mode so that multimodal and cross-polarization interference do not appear at the output arm. A change in the refractive index at the surface of the sensor arm results in an optical phase change on the sensing arm and a subsequent change in the light intensity measured at detector 130.

In an embodiment, the biomarker detection system 100 utilizes circularly polarized light to characterize and detect the target biomarker, based on the interaction of the circularly polarized light with the biomarker. Recent advances in optics have led to the notion of spinning electromagnetic fields capable of carrying angular momenta transverse to the direction of motion. Such fields enable numerous applications in nano-optics, biosensing and near-field microscopy, including three-dimensional control over atoms, molecules and nanostructures, and allowing for the realization of chiral nanophotonic interfaces and plasmonic devices. Broadband circular polarization conversion has been achieved with 3D optical metamaterials. An ideal material for nanophotonic applications such as those described herein may have a large refractive index at optical frequencies, respond to both the electric and magnetic fields of light, support large optical chirality and anisotropy, confine and guide light at the nanoscale, and be able to modify the phase and amplitude of incoming radiation in a fraction of a wavelength. Low-loss electromagnetic responses covering all four quadrants of possible permittivities and permeabilities have been achieved using completely transparent and high-refractive-index dielectric building blocks. These advances have revived the exciting prospect of integrating exotic electromagnetic effects in practical photonic devices, to achieve, for example, ultrathin and efficient optical elements, and realize the long-standing goal of subdiffraction confinement and guiding of light without metals. Manipulating the circular polarization of light is of great importance in chemistry and biology, as chiral molecules exhibit different physiological properties when exposed to different circularly polarized waves. The capability to manipulate the circular polarization of light is one of essential optical applications of molecular biology, medical science and analytical chemistry. Many biomolecules, including DNA, are chiral, which are sensitive to optical stimuli and thus behave differently when exposed to left-handed circular (LCP) waves and right-handed circular (RCP) waves. The use of superchiral electromagnetic fields represents a new approach to biospectroscopy and biosensing. In some cases, this phenomenon can be used to characterize minute amounts of a virus, and it may be possible to discriminate rapidly between isosahedral viruses (which usually have coat proteins with folds based on $\beta$-sheet structure) from cylindrical and filamentous viruses (which usually have $\alpha$-helical coat proteins folds). Circular dichroism (CD) is commonly observed in many chiral media, where right-handed (RH) and left-handed (LH) circularly polarized light exhibit different transmission when passing through the media. Previous research results have shown that circular polarization band gaps can be formed in a dielectric helix array not only by light having the same handedness with the structure but also by light with the opposite handedness, resulting from additional chiral motifs induced by the arrangement of helices. Dual polarization band gaps can thus be tailored by varying the geometrical parameters, and circular-polarization dependent properties can be manipulated for optoelectronic devices and applications.

Sensitivity can be important to consider when designing the biosensing element/waveguide. Generally speaking, there are two parts contributing to total sensitivity: waveguide sensitivity (SWG) and device sensitivity (Sd). Device sensitivity is the ratio of the change in the measured optical parameter (e.g. the resonance wavelength, or the intensity at a specific wavelength) to the change of the effective index. Waveguide sensitivity is defined as the ratio of the effective index change Dneff to the change Dns of the sample index (i.e. SWG = Dneff/Dns). In order to improve the sensitivity of the biosensing element/waveguide, one might improve the device design as well as the waveguide design. Device sensitivity depends mainly on the device structure, while SWG depends on the waveguide cross-section as well as the refractive index profile. One can optimize the device structure and the waveguide structure separately to maximize the device sensitivity and the waveguide sensitivity, respectively. Since only the evanescent field (which is a small part of the total guided-modal field) "experiences" the analyzed medium, the sensitivity SWG of a guided mode in an optical waveguide is usually assumed to be smaller or much smaller than that of a free-space beam (S=1). Silicon (Si) nanowires have become a favored choice because of their evanescent field enhancement in the cladding region due to the small cross section and the ultra-high index contrast. The molecular response of silicon photonic wire waveguides with silicon thicknesses in the range from 200 nm to 260 nm can be much higher than that of Surface Plasmon Resonance (SPR), due to the concentration of the evanescent field at the sensor surface, particularly for the TM polarized waveguide mode, and also because of the long optical propagation length possible in a sensor waveguide. TM polarization may provide increased sensitivity (e.g. SWG 0.5), though it is still less than that of a free-space beam (S=1).

In an embodiment comprising a biosensor module 110 having a plurality of biosensing elements 122 (e.g. a multiplexed biosensor chip), a 1500-1600nm laser light (e.g. a frequency swept laser) can be free space coupled into the input grating coupler on a first plate and transmitted to an input waveguide. The light then disperses from the input waveguide into multiple sensing "cells" that each comprise an input nanowire that splits into a reference arm and sensing arm with a Y junction that recombines the reference and sensing arms after a certain distance. In an embodiment, the reference arm and sensing arm comprise helical nanowire waveguides. The reference arm and sensing arm merge at an output waveguide, the output waveguide terminating at an output grating coupler of a second plate surface. In some variations, the output grating coupler is positioned at the bottom of the second plate. The output grating couple redirects light from the output waveguide to an off-module beam to array detector.

The recognition element may comprise any molecule suitable for binding the target biomarker; for example, the recognition element can be a biorecognition probe, a bioreceptor, an antibody, an enzyme, a DNAzyme, an aptamer, a cell etc. In some embodiments, the recognition element type and detection format used can include monoclonal antibodies, polyclonal antibodies (such as to pathogen-related protein antigens and human or animal disease-related protein biomarkers), antibody fragments, molecular imprinted polymers (MIPs) probe, nucleic acid/DNA/RNA probes (including aptamers), enzymatic probe, DNA methylation & histone post-translational modifications (epigenetics probe), cell-based probe, or other suitable label or detection method known in the art.

In one embodiment, the recognition element is spotted robotically on the sensor arm using a biofunctionalization process. In a particular case, the biofunctionalization process is noncontact piezoelectric-assisted inkjet bioprinting. This may be achieved using picoliter (pi) amounts of a specific antibody solution and automating the pipetting process. The robotic piezoelectric non-contact printer deposits dropsof solution containing the appropriate recognition element (diluted in phosphate-buffered saline (PBS) or other buffer of choice) on each exposed sensor arm in the array. An appropriate (pi) drop volume is chosen to fill the sensing area, (e.g. 500pl per 100um diameter) at an appropriate antibody concentration (e.g. can range from 1-1.5 mg/ml).

In alternative embodiments, direct-contact spotting methods may be used in addition to or in the place of non-contact methods. For example, a direct-contact spotting method may be used for recognition elements such as antibody fragments, molecular imprinted polymers (MIPs) probe, nucleic acid/DNA probe including aptamers, microRNAs, enzymatic probe, DNA methylation & histone post-translational modifications (epigenetics probe), cell-based probe.

In yet further embodiments, 3D bioprinting of individual recognition elements may be employed.

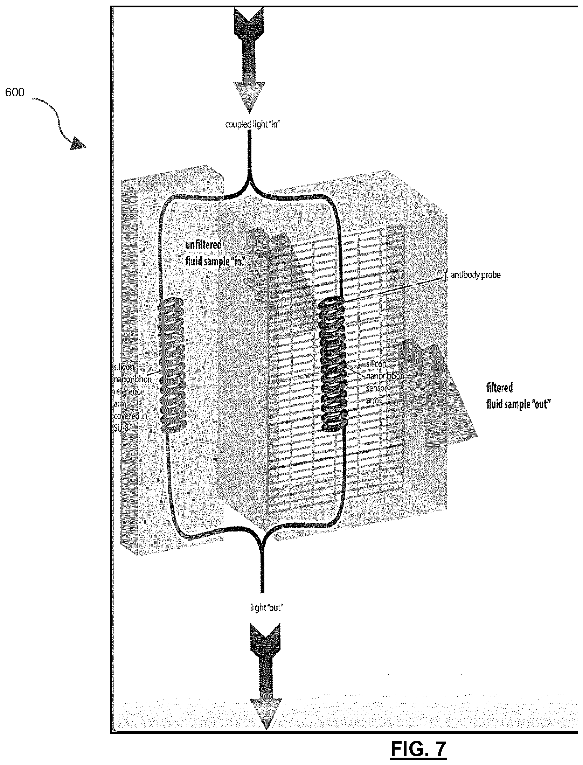

Referring now to FIG. 7, an individual sensor cell 700 having a bionsensing element for use in a biomarker detection system is shown, in accordance with an embodiment. The biosensing element of the individual sensor cell 700 comprises at least one waveguide, a beam splitter, and a beam combiner. The waveguide may have various segments having various functions, such as an input arm, a sensing arm (that is exposed to a nanofiltered sample), a reference arm, and an output arm. In a particular embodiment, the sensing arm is a helical coil sensing arm previously silanized and spotted with a recognition element for binding the biomarker present in the nanofiltered sample, and the reference arm is a helical coil covered in SU-8. In some cases, the waveguide may comprise multiple waveguides; for example, in a particular case the sensing arm and reference arm each comprise a waveguide, positioned between the input and output waveguides (i.e. between the two Y-junctions). Light from optical source 126 is coupled to the sensor cell 700, guided by the waveguide, and exits the cell 700 via the output arm to be received by detector 130.

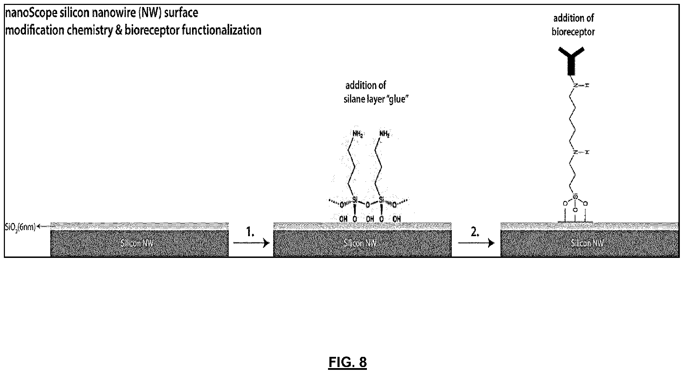

FIG. 8 shows a surface modification chemistry and recognition element functionalization for a silicon nanowire waveguide, in accordance with an embodiment. Cleaned silicon surfaces exposed in an ambient environment such as air or solutions may oxidize spontaneously. A silicon oxide (SiOx) layer comprising either native oxide or oxide grown under controlled conditions, passivates the surface of the silicon nanowire. This allows the silicon to be used as an electronic material in processing and application environments. After fabrication, or at least after exposure to ambient environment, the silicon nanowire comprises a SiOx layer. In some cases, the thickness of the SiOx layer on the nanowire may be approximately 1.5-10 nm, whereas the background oxide may be >10 nm.

In functionalizing the surface of the SiO2 surfaces of the silicon nanowire, silanization may be used. For example, 3-aldehydepropyltrimethoxysilane (APMS) or 3-aminopropyltriethoxysilane (APTES) may be applied as a way of surface modification to ensure covalent recognition element (e.g. monoclonal antibody) immobilization on the nanowire sensor arm. In a particular case, in the case of APMS, silicon waveguide nanowire sensing coils of the sensing arm comprising a passive oxide layer may undergo a reaction of 1% ethanol solution of 3-(trimethoxysilyl)propyl aldehyde for -30 min, washed with ethanol, and heated at 120°C for 15 minutes. This procedure may allow subsequent recognition elements that are to be spotted to be coupled to an aldehyde-terminated silicon nanowire surface.

Given its reactivity towards aldehyde, carboxylic acid and epoxy functionalities, APTES is a frequently used linker compound for further functionalization of silicon nanowires. The activation procedure of the SiOx nanowire surface using an oxygen plasma, followed by immersion in a solution of APTES in ethanol and subsequent heating of the biosensor module (or part thereof) may be used.

In the case of APTES, a silicon waveguide nanowire sensing coil having a passive oxide layer may be cleaned and activated using nitric acid and silanized with 3-aminopropyltriethoxysilane vapor in a vacuum chamber prior to recognition element spotting.

In some variations, surface passivation may also be incorporated. For example, a copolymer (e.g., poly(ethylene glycol)-b- poly(acrylic acid) (PEG-b-PAAc)) may be used as a blocking agent to reduce nonspecific binding on untreated and glutaraldehyde-activated regions. The limit of detection of such passivation may be down to 100 pM. The [biosensor module] may use dual polymers (i.e., PEG-b-PAAc and pentaethylenehex-amine-terminated PEG (N6-PEG)) on the same platform. Further, 1000-fold better sensitivity (100 fM) may be achieved with respect to blocking with PEG-b-PAAc.

Additional surface immobilization chemistry techniques (e.g. analyte capture efficiency, elimination of non-specific binding, etc.) may be used on the waveguide. In some embodiments, recognition element functionalization can include hydrosilylation on the silicon nanowire waveguide that make for oxide-free surfaces for the recognition element to bind. Other variations may include adding linear polyethylenimine-glutaraldehyde (LPEI-GA), which is a combination of noncovalent/covalent surface chemistry and which may demonstrate high stability.

Sensitivity can be important to consider when designing the biosensing element. Generally speaking, there are two parts contributing to the total sensitivity: the waveguide sensitivity (SWG) and the device sensitivity (Sd). The device sensitivity is the ratio of the change in the measured optical parameter (i.e., the resonance wavelength, or the intensity at a specific wavelength) to the change of the effective index. The waveguide sensitivity SWG is defined as the ratio of the effective index change Dneff to the change Dns of the sample index, i.e., SWG = Dneff/Dns. In order to improve the sensitivity of the biosensing element, one should improve the device design as well as the waveguide design. The device sensitivity Sd mainly depends on the device structure while the waveguide sensitivity SWG depends on the waveguide cross section as well as the refractive index profile. One can optimize the device structure and the waveguide structure separately to maximize the device sensitivity and the waveguide sensitivity, respectively. Since only the evanescent field (which is a small part of the total guided-modal field) "experiences" the analyzed medium, the sensitivity SWG of a guided mode in an optical waveguide is usually assumed to be smaller or much smaller than that of a free-space beam (S = 1). Si nanowires have become a favored choice because of their evanescent field enhancement in the cladding region due to the small cross section and the ultra-high index contrast. The molecular response of silicon photonic wire waveguides with silicon thicknesses in the range from 200 nm to 260 nm can be much higher than that of Surface Plasmon Resonance (SPR), due to the concentration of the evanescent field at the sensor surface, particularly for the TM polarized waveguide mode, and also because of the long optical propagation length possible in a sensor waveguide. TM polarization is usually used to have higher sensitivity, SWG 0.5, which, however, is still less than that of a free-space beam (S = 1).

In further variations on the present disclosure, structures and fabrications of MZI-related biosensors are contemplated, including: an increased interaction length of nanowire; utilizing a suspended nanowire configuration; manipulation of the polarization state of the optical waves traveling through the system, thereby enabling potential detection based on the interaction between chiral molecules and circularly polarized waves; incorporation of ultrathin metal surfaces prior to helical coil configuration; and helical coils acting as linear-to-circular polarization converters. With respect to chiral biomarkers, most biomolecules, including DNA, are chiral, which are sensitive to optical stimuli and thus behave differently when exposed to left-handed circular (LCP) waves and right-handed circular (RCP) waves. This phenomenon can be used to characterize minute amounts of a virus, and it may be possible to discriminate rapidly between isosahedral viruses (which usually have coat proteins with folds based on b-sheet structure) from cylindrical and filamentous viruses (which usually have a-helical coat proteins folds).

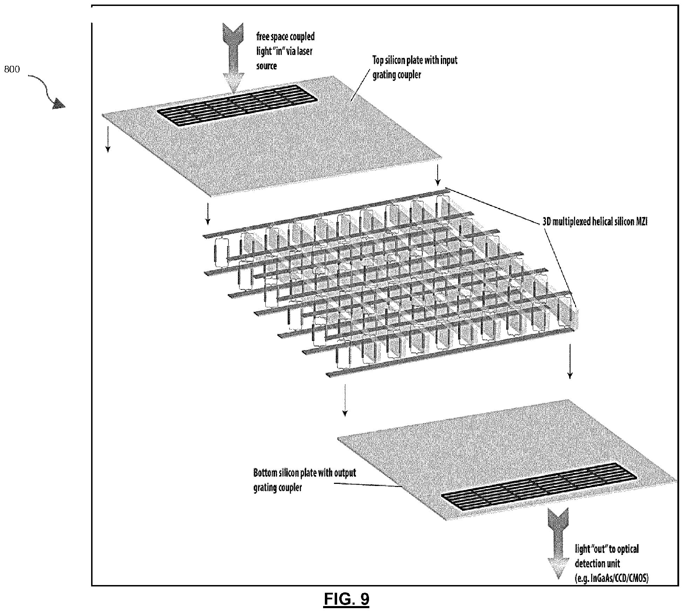

Referring to FIG. 9, an embodiment of a mulitplexed biosensor chip 900 is shown. The multiplexed bionsensor chip 900 comprises a grid of individual cells combined to form a 3D multiplexed MZI positioned between a first silicon plate and a second silicon plate, with the first plate having an input grating coupler and the second plate having an output coupler. The input grating coupler and output coupler may be fabricated by 3D printing techniques, nanoimprint lithography (NIL), or other fabrication techniques known in the art.

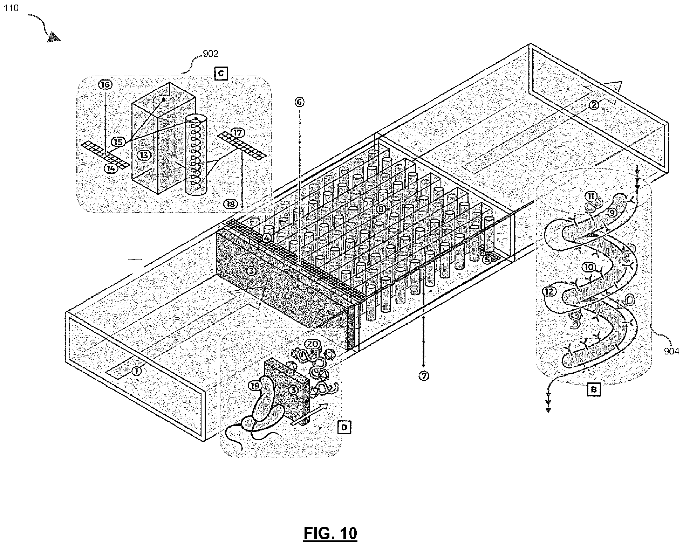

Referring now to FIG. 10, shown therein is biosensor module 110 comprising a plurality of biosensing elements 122, in accordance with an embodiment. The biosensor module 110 comprises a plurality of cells 1002, with each cell having a biosensing element 122. The biosensing element 122 comprises a waveguide having an input arm, an output arm, a reference arm, and a sensing arm 1004. In an embodiment, the reference arm is a silicon nanowire ribbon and the sensing arm 1004 is a coiled silicon nanowire treated with one or more biorecognition probes, such as an antibody. Coupled light enters an individual cell and travels via the input arm to a Y-junction, where the waveguide is split into the reference and sensing arms. The light is split at the Y-junction and travels down both reference and sensing arms and is recombined at the output arm where it exits the cell 1002 and may enter another individual cell or be read by detector 130.

The biosensor module 110 includes a filtration element for filtering the sample 102 prior to exposure to the biosensing elements 122. Light may be coupled to the biosensing elements 122 via an input grating coupler and output grating coupler. In some embodiments, at least a portion of the reference arm is covered by a cladding layer, preventing exposure to the sample 102.

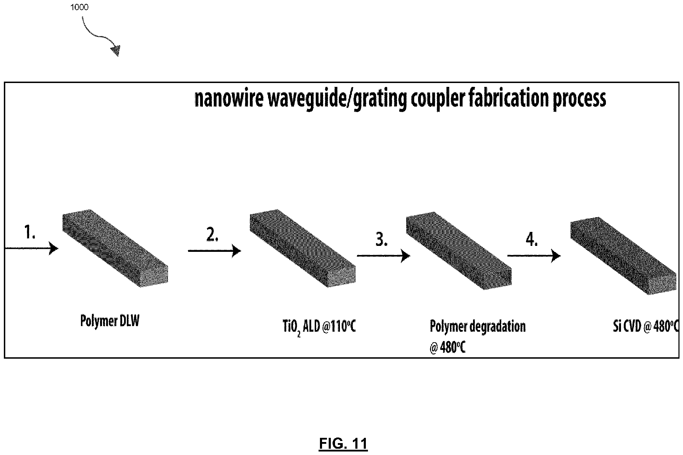

Referring now to FIG 11, a method 1100 of fabricating a nanowire waveguide with a grating coupler for use in a biomarker detection system/operation is shown, in accordance with an embodiment. First, direct laser writing (DLW) into a liquid photoresist is used to create polymer templates of the waveguides. Next, in order to mechanically stabilize the structures at higher temperatures, the polymer structure is coated with a thin $TiO_2$ layer by depositing a film of $TiO_2$ (e.g. roughly 8nm) at a moderate temperature of 110 °C using atomic-layer deposition (ALD). Next, the polymer is thermally degraded by keeping the sample at a temperature of 480 °C for about 20-30 min. Next, chemical vapor deposition (CVD) at 480 °C is used to infiltrate the ultralow density $TiO_2$ network structure with silicon.

Further embodiments contemplated utilize processes such as glancing angle deposition (GLAD) or nanoimprint lithography for the selective patterning of coiled silicon wire fabrication.

One or more of the processes of direct laser writing, GLAD, and NIL, described herein may also be used to fabricate input and output grating couplers on the first and second plates of the biosensor module 110. In an embodiment, the input and output grating couplers are printed on silicon plate substrate.

The detector 130 can be any suitable photodetector, and in some cases, may be a free-space detector. In a particular case, the detector 130 can be a balanced photodetector, which can have an increased signal to noise ratio. In further cases, the detector 130 may be a photoelectric-type photodetector, such as a charge-coupled device (CCD) or complementary metal-oxide semiconductor (CMOS). The detector 130 may operate by photoemission, photovoltaic, thermal, photochemical, or polarization mechanism, or other mechanism through which electromagnetic energy can be converted into an electrical signal. Upon receiving the light, the detector 130 can convert the radiance/intensity of the light into an electrical signal. In some cases, the electrical signal may then be converted to a digital signal, and modified by signal conditioning techniques such as filtering and amplification. In some cases, an interference pattern corresponding to the path of the light can be converted into a signal by the detector 130 via, for example, a high-speed digitizer. The signal received by detector 130 can be converted to a digital signal by a photonic analog-digital converter. The digital signal can then have signal processing functions and techniques applied to it by the computing module 134.

In some cases, the detector 130 may comprise a spectrometer. In such cases, the system 100 may operate by interrogating the sample 102 with one or more specific wavelengths of electromagnetic radiation via optical source 126 and by detecting the resulting emission or scattering of energy produced as detected spectral signals. By analyzing the range of amplitudes and wavelengths in the detected spectral signals, components in the sample 102 including the target biomarker can be identified. The optical source 126 may output radiation in the visible, near infrared or near ultraviolet range. The output radiation can interact with molecular vibrations or other excitations in the sample 102, resulting in the energy of the photons in the output radiation being changed.

As previously described, the biomarker detection system 100 also includes a computing module 134. The computing module 134 may be locally communicatively linked or remotely communicatively linked, for example via network 138, to one or more other elements of the system 100; for example, to the optical source 126, the detector 130, the microfluidic device 114, the biosensor module 110. The computing module 134 may be used for processing and analysis of detection signals (and data derived therefrom) provided by the biomarker detection system 100. In some cases, the computing module 134 may operate as a control system or controller, and in other cases, may be connected to a separate control system or controller. Further, the computing module 134 may host a user-accessible platform for invoking services, such as reporting and analysis services, and for providing computational resources to effect machine learning techniques on the detection data. Where appropriate, computing module 134 may include one or more computing modules 134; be unitary or distributed; span multiple locations; span multiple machines; or reside in a cloud, which may include one or more cloud components in one or more networks. Where appropriate, one or more computing modules 134 may perform without substantial spatial or temporal limitation one or more steps of one or more methods described or illustrated herein. One or more computing modules 134 may perform at different times or at different locations one or more steps of one or more methods described or illustrated herein, where appropriate. The computing modules 134 can include clients and servers. A client and server are generally remote from each other and typically interact through network 138. The relationship of client and server arises by virtue of computer programs running on the respective computers and having a client-server relationship to each other.

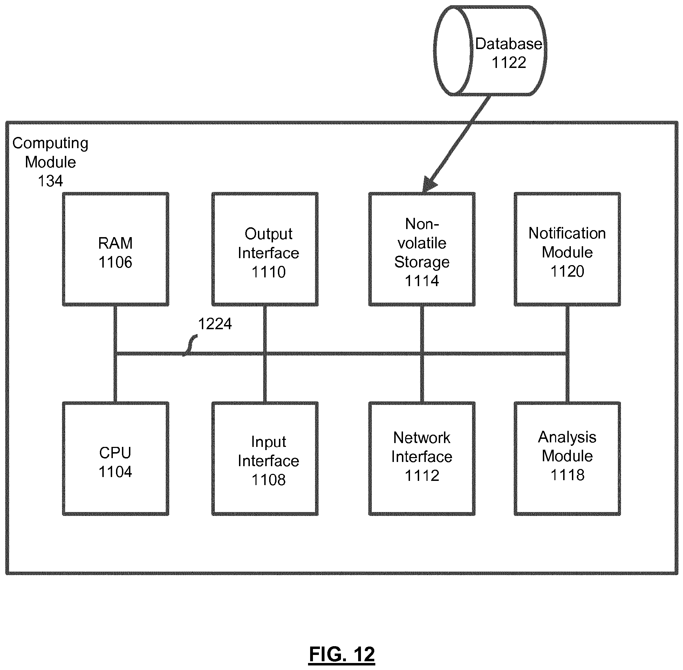

In an embodiment, as shown in FIG. 12, the computing module 134 can include a number of physical and logical components, including a central processing unit ("CPU") 1204, random access memory ("RAM") 1206, an input interface 1208, an output interface 1210, a network interface 1212, non-volatile storage 1214, and a local bus 1224 enabling CPU 1204 to communicate with the other components. CPU 1204 can include one or more processors. RAM 1206 provides relatively responsive volatile storage to CPU 1204. The input interface 1208 enables an administrator to provide input via, for example, a keyboard and mouse. The output interface 1210 outputs information to output devices, for example, a display or speakers. The network interface 1212 permits communication with other systems or computing devices. Non-volatile storage 1214 stores the operating system and programs, including computer-executable instructions for implementing the biomarker detection system 100 or analyzing data from the biomarker detection system 100, as well as any derivative or related data. In some cases, this data can be stored in a database 1222. During operation of the system 100, the operating system, the programs and the data may be retrieved from the non-volatile storage 1214 and placed in RAM 1206 to facilitate execution. In an embodiment, the CPU 1204 can be configured to execute various modules, for example, an analysis module 1218 and a notification module 1220. In some cases, the computing module 134, via for example the analysis module 1218, can use machine learning (ML) to transform raw data from the detection signal into a descriptor. The descriptor may be information associated with a particular biomarker in the sample. The descriptor can then be used to determine a classifier for the biomarker. As an example, the CPU 1204 may do this detection and classification with auto-encoders as part of a deep belief network.

Analysis of detection signals by computing module 134 can be based on various methods. For example, two different reference signals can be identified, the first one indicating the absence and the second one the presence of the desired substance. Accordingly, signals obtained for each sample 102 can be compared to the reference signals, and a determination can be made as to whether the desired substance is present in the sample. The determination can then be indicated to a user of the device.

As a non-limiting illustration, in the present example, the sample 102 includes the specified biomarkers as the desired substance and the specified antibodies. The combined sample 102 of the present example can result in two potentially different spectral signals which can be identified as the two reference signals. A first reference signal results from antibodies that are not bound to the biomarkers. A second reference signal results from the antibodies that are bound to the specified biomarkers. Accordingly, when spectral signals obtained from a combined sample 102 are analyzed, a determination can be made as to whether the combined sample 102 includes biomarkers or whether the biomarkers are absent. Specifically, the obtained spectral signals can be compared with the two reference signals to make a determination. The determination that the sample 102 includes the biomarkers, in turn, indicates the presence of specified biomarker with the sample provider.

The identification of the reference signals, which can be more than two or less than two, depending on the detection technique or techniques used, can be based on detection signals and various methods. In some variations, the identification can be made manually, by performing the detection operations on the sample 102 in the presence and absence of the desired substance, and selecting the appropriate signals as reference signals. In variations, the identification can be made automatically based on various automated learning algorithms such as supervised, semi-supervised and unsupervised learning algorithms, through the use of neural networks or clustering mechanism, for example. Neural networks used can be probabilistic. In some variations, the same mechanisms used for automatically identifying the reference signals can also be used to perform the signal match analysis. For example, neural networks or clustering mechanism used for identifying reference signals can also be used for performing the matching of a detected signal to one of the reference signals. In yet further variations, there may not be separate reference signal identification process. Instead, learning based mechanisms, such as neural networks and clustering mechanism can learn to detect the presence or absence of a desired substance based on the detected signals, employing various learning schemes. In some further variations, the learning mechanisms can be primed with unsupervised data so that they are primed for the detection of the presence or absence of the desired substance based on detection signals received from the detector 130.

In variations, the detection or quantification of a biomarker in the sample using a neural network or clustering mechanism can be an ongoing process. For example, in some implementations, the computing module 134 can be a local computing module and provide results to a remote computing module, such as remote computing module 142 of system 100. The remote computing module 142 can include appropriate learning mechanisms to update a training model based on the newly received signals. For example, the remote computing module can be a neural network based system implemented using various application programming interfaces APIs and can be a distributed system. The APIs included can be workflow APIs, match engine APIs, and signal parser APIs, allowing the remote computing module to both update the network 138 and to determine whether a biomarker is present or absent based on the received detection signal.

In further variations, the analysis of the detection signals as well as identification of the reference signals can include additional data obtained from sources other than the detector 130. For example, vital sign measurements (eg. heart rate, body temperature, respiratory rate, blood pressure, SpO2), thermal imaging signals, sample provider history, computerized adaptive tests, inventories and medical imaging (eg. CT scan, neuroimaging, ultrasonography, mammography, MRI, chest x-ray, colonoscopy, digital pathology analysis) and other data can be included in the analysis (and, where appropriate as part of the reference signal), in addition to the detection signals from the detector 130. For example, in the present example, when the detection signal for a sample 102 is matched to a reference signal indicating the presence of the specified biomarker with a weak confidence level, the above examples may be used, for example a thermal image indicating a fever may be used to increase the confidence level of the match. As a further example, the detection signal can include travel pattern of the sample provider, thus allowing the system 100 to take into account the sample provider's travel history in determining a match, and thus the presence or absence of the desired substance.

Embodiments of the systems and methods of the present disclosure may implement groundtruthing to ensure classification result accuracy according to an active learning technique. Specifically, results from classification models may be rated with a confidence score, and high uncertainty classification results can be pushed to a groundtruther to verify classification accuracy. Optionally, classification outputs can periodically be provided to groundtruthers to ensure accuracy. In some implementations, a determination by the system indicative of detection of a particular biomarker of interest may result in generating a request for human groundtruthing of the detection signal or the target object from which the detection signal was generated.

In some implementations, the method for detecting a desired substance can be varied such that multiple desired substances can be detected. More complex analysis, such as those based on clustering methods and neural networks can also be used to differentiate between the different substances based on one or more detection signals obtained on the basis of a sample 102.

In the following, machine learning implementations of the systems and methods described above will be described in additional detail.

In some embodiments described above, neural networks data analysis may be utilized for detecting or quantifying a specified biomarker based on the detected signals, employing various learning techniques. These embodiments may be carried out by a processor of computing module 134, or by remote computing module 142 in communication with computing module 134 over the network 138, optionally during the analysis of detection signals generated by the detector 130 during the course of a detection operation. As described above, detection signals may be received from a detector 130 at the computing module 134.

Analysis may be implemented by providing input data to a neural network, such as a feed-forward neural network, for generating at least one output. The neural networks described below may have a plurality of processing nodes, including a multi-variable input layer having a plurality of input nodes, at least one hidden layer of nodes, and an output layer having at least one output node. During operation of a neural network, each of the nodes in the hidden layer applies a function and a weight to any input arriving at that node (from the input layer or from another layer of the hidden layer), and the node may provide an output to other nodes (of the hidden layer or to the output layer). The neural network may be configured to perform a regression analysis providing a continuous output, or a classification analysis to classify data. The neural networks may be trained using supervised or unsupervised learning techniques, as described above. According to a supervised learning technique, a training dataset is provided at the input layer in conjunction with a set of known output values at the output layer. During a training stage, the neural network may process the training dataset. It is intended that the neural network learn how to provide an output for new input data by generalizing the information it learns in the training stage from the training data. Training may be effected by backpropagating error to determine weights of the nodes of the hidden layers to minimize the error. The training dataset, and the other data described herein, can be stored in a database connected to the computing module 134 or otherwise accessible to computing module 134 or remote computing module 142. Once trained, or optionally during training, test data can be provided to the neural network to provide an output. A neural network may thus cross-correlate inputs provided to the input layer in order to provide at least one output at the output layer. Preferably, the output provided by a neural network in each embodiment will be close to a desired output for a given input, such that the neural network satisfactorily processes the input data.

According to an embodiment, a neural network interprets received detection signals from detector 130. The neural network may be configured as a convolutional feed-forward neural network. Accordingly, during use, at least a measured detection signal, or some scaled or otherwise modified value thereof, can be provided to the neural network as an input [at the input layer]. Optionally, additional data may be provided to the input layer of the neural network to assist in interpreting received detection signals from detector 130. For example, combinations of data could be provided at the input layer, including: protein interaction data, and genomic/nucleic acid data, subject and/or specific substance (e.g. biomarker). In such cases, high-throughput genomic sequencing of the subject may be required, and may be performed by remote computing module 142 and need not be carried out at computing module 134. Other input data may include mass spectrometry data (e.g. from protein sequencing), time series genomic data of the condition of interest and subject history (e.g. medical history). Embodiments may thus cross-correlate various inputs to provide an output to aid in interpreting a detection signal to determine whether the biomarker has been detected. In some cases, this additional data may be received from a third-party data repository.

An output indicating biomarker presence in the sample 102 may result in a notification being generated by computing module 134 (such as via notification module 1220) and sent (as an alert/notification) to a medical professional; the medical professional may be local, already associated with the patient, or an expert in the healthcare field with special knowledge of the disorder to which the biomarker is linked. Further, a positive output may result in computing module 134 generating a request for human ground-truthing of the detection signal or sample 102. For example, a microscopic image of sample 102 can be electronically transmitted from computing module 134 to the ground truther for assessment. Further, a notification may be sent (e.g. via notification module 1220) to the subject advising of any actions (immediate or otherwise) that should be taken for their own health and safety or the health and safety of others.

In another embodiment, a neural network is applied to compensate for nanoscale and quantum realm detection limitations. Particularly, a detection signal is provided to a neural network at the input layer, with a desired output compensating for defects in the detection signal that may be caused by limitations of imaging in the nano-realm. The input layer may receive data relating to the detection modality and an input detection signal for detection of viruses, bacteria, fungi, parasites, human host (i.e. subject) cells, disease biomarkers, etc. The neural network may be trained such that the output layer provides a clean detection signal compensating for signal defects. Particularly, the neural network may be trained with a training dataset comprising, at the input layer, detection signals comprising nano-realm defects, and with associated clean detection signals at the output layer for viruses, bacteria, fungi, parasites, human host cells, disease biomarkers, etc. for detection modalities. The output of the trained neural network may provide a processed detection signal similar to known reference signals for particular detection modalities such that processing by the neural network remedies some defects and limitations of received detection signals.

In another embodiment, a neural network-based predictive output machine is provided. Particularly, the machine learning predictive output machine may receive inputs comprising time series genomic data of a subject in order to provide an output indicative of a clinical outcome. To provide time series inputs, samples may be taken and sequenced from a subject having a condition of interest over a period of time to maintain or improve the accuracy of the neural network over time. To train the neural network, a training dataset may comprise known inputs of the specified data types as well-known associated clinical outcomes. Further data inputs may include time series genomic data of a condition of interest, subject history (e.g. medical history), and subject condition (e.g. resistivity to a particular infection or disease).

The present disclosure teaches a system, method, and module for detecting presence or absence of a biomarker in a sample.

The above described embodiments of the present disclosure are intended to be examples of the present disclosure and alterations and modifications may be effected thereto, by those of skill in the art, without departing from the scope of the present disclosure, which is defined solely by the claims appended hereto. For example, systems, methods, and embodiments discussed can be varied and combined, in full or in part.

Thus, specific systems, methods, and modules for detecting a biomarker in a sample have been disclosed. It should be apparent, however, to those skilled in the art that many more modifications besides those already described are possible without departing from the inventive concepts herein. The subject matter of the present disclosure, therefore, is not to be restricted except in the spirit of the disclosure. Moreover, in interpreting the present disclosure, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

Systems, methods, and modules for detecting a biomarker in a sample are described. A system for detecting presence or absence of a biomarker in a sample includes: a light source for producing electromagnetic radiation for interrogating the sample; a biosensor module including: a waveguide for guiding the electromagnetic radiation, the waveguide exposed to the sample; and a recognition element affixed to the waveguide and configured to bind to the biomarker; a detector for receiving the electromagnetic radiation from the waveguide and detecting a signal corresponding to an interaction of the electromagnetic radiation with the biomarker bound to the recognition element, in accordance with at least one detection modality; and a computing device for analyzing data related to the signal in order to detect presence or absence of the biomarker in the sample.

**Claims**

1.    A system for detecting presence or absence of a biomarker in a sample comprising:

a light source for producing electromagnetic radiation for interrogating the sample;
a biosensor module comprising:

a waveguide for guiding the electromagnetic radiation, the waveguide exposed to the sample; and
a recognition element affixed to the waveguide and configured to bind to the biomarker;

a detector for receiving the electromagnetic radiation from the waveguide and detecting a signal corresponding to an interaction of the electromagnetic radiation with the biomarker bound to the recognition element, in accordance with at least one detection modality; and
a computing device, having one or more processors, for analyzing data related to the signal in order to detect presence or absence of the biomarker in the sample.

2.    The system of claim 1, further comprising a microfluidic device for effecting movement of the sample through the biosensor module.

3.    The system of claim 1, wherein at least one of the detection modalities is an interferometry modality for generating an interference pattern as the signal.

4. The system of claim 1, wherein at least one of the detection modalities is selected from the group consisting of Surface-Enhanced Raman Spectroscopy (SERS), Surface Plasmon Resonance (SPR), Surface Plasmon Resonance Imaging (SPRi), Localised Surface Plasmon Resonance (LSPR), Optofluidic Nanoplasmonic, Optical waveguide-based sensing, Optical ring resonator-based sensing, Photonic crystal-based sensing, Nanosensitive Optical Coherence Tomography (OCT) sensing, Lensless digital holographic imaging, Superresolution microscopy techniques, piezoelectric sensing, nano-cantilever sensing, Raman spectroscopy (RS), Resonance Raman spectroscopy (RRS), and infrared spectroscopy (IRS).

5. The system of claim 1, wherein the computing device comprises a neural network for receiving the data related to the signal at an input layer and generating the determination of the presence or absence of the biomarker in the sample at an output layer.

6. The system of claim 5, wherein data received at the input layer further comprises at least one of protein interaction data, nucleic acid data, biomarker identification data, genomic sequencing data, mass spectrometry data, time series genomic data, and medical history data.

7. The system of claim 1, wherein the biosensor module comprises a plurality of cells, each cell comprising a separate waveguide and recognition element.

8. The system of claim 1, wherein the waveguide couples the electromagnetic radiation using a coupling approach selected from the group consisting of front-face coupling, prism coupling, and grating coupling.

9. A method of detecting presence or absence of a biomarker in a sample, comprising:

exposing a waveguide to the sample and binding the biomarker via a recognition element affixed to the waveguide;
producing electromagnetic radiation directed at the waveguide, the waveguide guiding the electromagnetic radiation towards a detector;
receiving the electromagnetic radiation at the detector;
detecting a signal at the detector corresponding to the interaction of the electromagnetic radiation with the biomarker bound to the recognition element, in accordance with at least one detection modality; and
determining presence or absence of the biomarker, by a computing device having one or more processors, by analyzing data related to the signal.

10. The method of claim 9, wherein at least one of the detection modalities is an interferometry modality for generating an interference pattern as the signal.

11. The method of claim 9, wherein at least one of the detection modalities is selected from the group consisting of Surface-Enhanced Raman Spectroscopy (SERS), Surface Plasmon Resonance (SPR), Surface Plasmon Resonance Imaging (SPRi), Localised Surface Plasmon Resonance (LSPR), Optofluidic Nanoplasmonic, Optical waveguide-based sensing, Optical ring resonator-based sensing, Photonic crystal-based sensing, Nanosensitive Optical Coherence Tomography (OCT) sensing, Lensless digital holographic imaging, Superresolution microscopy techniques, piezoelectric sensing, nano-cantilever sensing, Raman spectroscopy (RS), Resonance Raman spectroscopy (RRS), and infrared spectroscopy (IRS).

12. The method of claim 9, wherein determining presence or absence of the biomarker comprises using a neural network for receiving the data related to the signal at an input layer and generating the determination of the presence or absence of the biomarker in the sample at an output layer.

13. The method of claim 12, wherein the neural network is a convolutional feed-forward neural network.

14. The method of claim 12, wherein data received at the input layer further comprises at least one of protein interaction data, nucleic acid data, biomarker identification data, genomic sequencing data, mass spectrometry data, time series genomic data, and medical history data.

15. The method of claim 9, wherein the waveguide couples the electromagnetic radiation using a coupling approach selected from the group consisting of front-face coupling, prism coupling, and grating coupling.

**FIG. 1**

200

Between cheek and gums

Under the front of the tongue

1. Insert Swab into the syringe cavity

Salimetrics Oral Swab

5 cc Syringe

2. Use plunger to express sample into a vial

Salimetrics Oral Swab

**FIG. 2**

200

Connect to pressure pump

Test sample

Waste container

Wash buffer

Optical sensing area with
suspended multiplexed
coiled nanowire waveguide

**FIG. 3**

300

The illustration of self-powered chip with washing step

FIG. 4

400

(a)

Threads

Solution Reservoir

(b) Withdrawing

Pumping

(a)

Handling head

Channels

**FIG. 5**

FIG. 6

600

coupled light "in"

unfiltered
fluid sample "in"

Y antibody probe

silicon
nanoribbon
reference
arm
covered in
SU-8

silicon
nanoribbon
sensor
arm

filtered
fluid sample "out"

light "out"

FIG. 7

nanoScope silicon nanowire (NW) surface
modification chemistry & bioreceptor functionalization

addition of
silane layer "glue"

addition of
bioreceptor

**FIG. 8**

free space coupled
light "in" via laser
source

Top silicon plate with input
grating coupler

800

3D multiplexed helical silicon MZI

Bottom silicon plate with output
grating coupler

light "out" to optical
detection unit
(e.g. InGaAs/CCD/CMOS)

**FIG. 9**

**FIG. 10**

1000

## nanowire waveguide/grating coupler fabrication process

1. → Polymer DLW

2. → $TiO_2$ ALD @110°C

3. → Polymer degradation @ 480°C

4. → Si CVD @ 480°C

FIG. 11

**FIG. 12**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/053147 A1 (OSTENDUM HOLDING B V [NL]; YMETI AUREL [NL]; NEDERKOORN PAULUS HENDRIC) 5 May 2011 (2011-05-05)<br>* page 1, line 28 - page 2, line 23 *<br>* page 4, lines 19-31 *<br>* page 13, line 32 - page 14, line 36 *<br>* page 15, lines 30-32 *<br>* page 16, line 34 - page 19, line 6 *<br>* page 23, line 20 - page 24, line 27 *<br>* page 34, line 31 - page 35, line 14 *<br>* figures 1, 3, 4, 8 *<br>----- | 1-3,9,10 | INV.<br>G01N21/77<br><br>ADD.<br>G01N21/35<br>G01N21/552<br>G01N21/65 |
| X | US 5 623 561 A (HARTMAN NILE F [US]) 22 April 1997 (1997-04-22)<br>* column 8, lines 32-62 *<br>* column 10, lines 21-47 *<br>* column 14, lines 5-24 *<br>* column 16, lines 56-60 *<br>* figures 1, 3A-3C, 5 *<br>----- | 1,3, 7-10,15 | |
| X | FAN X ET AL: "Sensitive optical biosensors for unlabeled targets: A review",<br>ANALYTICA CHIMICA ACTA, ELSEVIER, AMSTERDAM, NL,<br>vol. 620, no. 1-2,<br>14 July 2008 (2008-07-14), pages 8-26, XP022732615,<br>ISSN: 0003-2670, DOI:<br>10.1016/J.ACA.2008.05.022<br>[retrieved on 2008-06-13]<br>* pages 13-18 *<br>----- | 1,3,4, 8-11,15 | TECHNICAL FIELDS SEARCHED (IPC)<br><br>G01N |
| X | US 2012/212732 A1 (SANTORI CHARLES M [US] ET AL) 23 August 2012 (2012-08-23)<br>* paragraphs [0027], [0033], [0034], [0037], [0038], [0065] *<br>* figures 2, 10 *<br>-----<br>-/-- | 1,4,9,11 | |

The present search report has been drawn up for all claims

| Place of search | Date of completion of the search | Examiner |
|---|---|---|
| Munich | 16 November 2017 | Hoogen, Ricarda |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 485 277 A (FOSTER MARK W [US]) 16 January 1996 (1996-01-16) <br> * column 8, lines 35-61 * <br> * column 9, lines 34-41 * <br> * column 17, lines 28-34 * <br> * figures 4(a), 4(b) * | 1,4,9,11 | |
| X | SKIVESEN N ET AL: "Photonic Crystal Waveguide-based Biosensor", OPTICAL FIBER COMMUNICATION/NATIONAL FIBER OPTIC ENGINEERS CONFERENCE, 2008. OFC/NFOEC 2008. CONFERENCE ON, IEEE, PISCATAWAY, NJ, USA, 24 February 2008 (2008-02-24), pages 1-3, XP031391732, ISBN: 978-1-55752-856-8 <br> * the whole document * | 1,4,9,11 | |
| X | KIRILL ZINOVIEV ET AL: "Optical biosensor based on arrays of waveguide microcantilevers", PROCEEDINGS OPTICAL DIAGNOSTICS OF LIVING CELLS II, vol. 6477, 6 February 2007 (2007-02-06), page 64771A, XP55425540, US ISSN: 0277-786X, DOI: 10.1117/12.698176 ISBN: 978-1-5106-1354-6 <br> * the whole document * | 1,4,9,11 | |
| X | US 2016/122794 A1 (TRENHOLM WALLACE [CA] ET AL) 5 May 2016 (2016-05-05) <br><br> * paragraphs [0034], [0038], [0040], [0061], [0068], [0069], [0107] - [0109] * <br> * figure 18 * | 1,2,5,6, 8,9, 12-15 | TECHNICAL FIELDS SEARCHED (IPC) |

The present search report has been drawn up for all claims

| Place of search | Date of completion of the search | Examiner |
|---|---|---|
| Munich | 16 November 2017 | Hoogen, Ricarda |

EPO FORM 1503 03.82 (P04C01)

## ANNEX TO THE EUROPEAN SEARCH REPORT
## ON EUROPEAN PATENT APPLICATION NO.

EP 17 17 8959

This annex lists the patent family members relating to the patent documents cited in  the above-mentioned European search report.
The members are as contained in the European Patent Office EDP file on
The European Patent Office is in no way liable for these particulars which are merely given for the purpose of information.

16-11-2017

| Patent document cited in search report | | Publication date | Patent family member(s) | | Publication date |
|---|---|---|---|---|---|
| WO 2011053147 | A1 | 05-05-2011 | CN | 102713578 A | 03-10-2012 |
| | | | EP | 2496930 A1 | 12-09-2012 |
| | | | JP | 5657013 B2 | 21-01-2015 |
| | | | JP | 2013509581 A | 14-03-2013 |
| | | | NL | 2003743 C | 06-04-2011 |
| | | | US | 2012214707 A1 | 23-08-2012 |
| | | | WO | 2011053147 A1 | 05-05-2011 |
| US 5623561 | A | 22-04-1997 | AT | 217081 T | 15-05-2002 |
| | | | AT | 322676 T | 15-04-2006 |
| | | | AU | 7377096 A | 17-04-1997 |
| | | | CA | 2233305 A1 | 03-04-1997 |
| | | | DE | 69621031 D1 | 06-06-2002 |
| | | | DE | 69621031 T2 | 22-08-2002 |
| | | | DE | 69636019 T2 | 29-03-2007 |
| | | | DK | 0852715 T3 | 19-08-2002 |
| | | | EP | 0852715 A1 | 15-07-1998 |
| | | | EP | 1182445 A2 | 27-02-2002 |
| | | | ES | 2172683 T3 | 01-10-2002 |
| | | | JP | 3924211 B2 | 06-06-2007 |
| | | | JP | H11505023 A | 11-05-1999 |
| | | | JP | 2003121675 A | 23-04-2003 |
| | | | PT | 852715 E | 31-10-2002 |
| | | | US | 5623561 A | 22-04-1997 |
| | | | WO | 9712225 A1 | 03-04-1997 |
| US 2012212732 | A1 | 23-08-2012 | NONE | | |
| US 5485277 | A | 16-01-1996 | NONE | | |
| US 2016122794 | A1 | 05-05-2016 | CA | 2966215 A1 | 06-05-2016 |
| | | | EP | 3213081 A1 | 06-09-2017 |
| | | | US | 2016122794 A1 | 05-05-2016 |
| | | | WO | 2016065487 A1 | 06-05-2016 |

EPO FORM P0459

For more details about this annex : see Official Journal of the European Patent Office, No. 12/82